# EUROPEAN PATENT APPLICATION

(11) **EP 4 803 013 A1**
(43) Date of publication of application: **09.09.2026**
(21) Application number: 25161229.7
(22) Date of filing: 03.03.2025
(51) Int. Cl.: A61B 8/08, A61B 8/00, G16H 30/40, G16H 50/30

(54) **PROCESSING FOUR-CHAMBER VIEW ULTRASOUND IMAGE DATA**

(71) Applicant: Koninklijke Philips N.V., 5656 AE Eindhoven (NL)
(72) Inventor: AWASTHI, Manish, Eindhoven (NL); SUBASH CHANDRAN, Dheepak, Eindhoven (NL); NELLUR PRAKASH, Sindhu Priyadarshini, 5656AG Eindhoven (NL); VELUMURGAN, Mylavan, Eindhoven (NL); SHETTIGAR, Srikanth, Eindhoven (NL); FIRTION, Celine, Eindhoven (NL); NANJUNDEGOWDA, Navya, Eindoven (NL); VAJINEPALLI, Pallavi, Eindhoven (NL)
(74) Representative: Philips Intellectual Property & Standards

(57) **Abstract**

A mechanism for processing four-chamber ultrasound image data. The image data is processed to predict a cardiac phase represented by the view by first identifying an atrial measure and a ventricle measure. These measures are subsequently processed to identify the cardiac phase.

## Description

### FIELD OF THE INVENTION

The present invention relates to the field of processing of cardiac ultrasound image data.

### BACKGROUND OF THE INVENTION

There is an increasing reliance upon medical imaging systems for aiding a clinician to assess the condition of a subject. One example of a medical imaging system is an ultrasound imaging system, which produces ultrasound image data (e.g., one or more ultrasound (digital) images).

One particular use case for ultrasound image data is in echocardiography, particularly fetal heart screening. In such examples, cardiac ultrasound image data for different views of the fetal heart is captured and processed, e.g., to perform anatomical measurements and/or pathological analysis to identify any potential anomalies. However, other use-case scenarios for cardiac image data are known in the art (e.g., to assess the condition of an adult heart).

In cardiac image analysis, different viewing planes are commonly used to investigate or identify any anomalies, if present. One particularly useful viewing plane is the four-chamber (4CH) view, which is provided by four-chamber ultrasound image data. As the heart is a dynamic organ, it has different phases which can be used for different anatomical measurements and/or pathological analysis during different phases of heart cycle to identify or rule out different heart conditions (such as congenital heart diseases (CHDs)).

There is an ongoing desire to facilitate improved analysis of cardiac ultrasound image data, and in particular four-chamber ultrasound image data.

### SUMMARY OF THE INVENTION

The invention is defined by the claims.

In accordance with a proposed approach, there is provided a computer-implemented method of processing four-chamber ultrasound image data, providing a four-chamber view of a heart of a subject. The method comprises: processing the four-chamber ultrasound image data to determine an atrial measure providing a measure of a size of atria represented in the four-chamber ultrasound image data; processing the four-chamber ultrasound image data to determine a ventricle measure providing a measure of a size of ventricles represented in the four-chamber ultrasound image data; and processing the atrial measure and the ventricle measure to predict a cardiac phase of a cardiac cycle of the subject represented by the four-chamber ultrasound image data.

The present disclosure provides a mechanism for determining or predicting in which phase of a cardiac cycle the four-chamber ultrasound image data was captured. In particular, it has been recognized that the size of the atria and ventricles are indicative of the cardiac phase of the heart when the ultrasound image data was captured. In this way, an automatic mechanism for predicting the cardiac phase is achieved.

In some examples, the processing the atrial measure and the ventricle measure comprises: determining a ratio between the atrial measure and the ventricle measure; and processing the determined ratio to predict the cardiac phase of the cardiac cycle of the subject represented by the four-chamber ultrasound image data.

This embodiment recognizes that, in particular, the relative size between the atria and the ventricles of the heart is indicative or changes responsive to the particular phase of the cardiac cycle in which the ultrasound image data was captured. This provides a more accurate approach for predicting the cardiac phase.

Moreover, this reliance on relative chamber sizes rather than absolute measurements may make it more resilient to variations in image quality or differences in ultrasound equipment, capture modalities and/or subject variations. This provides a more widely applicable mechanism for use in different clinical settings.

In some examples, the processing the determined ratio comprises: identifying which of a plurality of predefined ranges bounds the determined ratio; and predicting the cardiac phase of the cardiac cycle of the subject responsive to the identified predefined range. This approach recognizes that different ranges of the ratio between atrial and ventricular sizes corresponds to specific phases of the cardiac cycle. By categorizing the calculated ratio into predefined ranges, the method is able to more efficiently predict the cardiac phase, e.g., without needing to rely upon complex computational models or extensive training data.

Preferably, the plurality of predefined ranges covers all possible values for the ratio. Preferably, the plurality of predefined ranges are non-overlapping.

In some examples, each predefined range is associated with a different phase of the cardiac cycle of the subject; and the predicting the cardiac phase comprises identifying the phase of the cardiac cycle associated with the identified predefined range.

In some examples, the plurality of predefined ranges comprises at least: a first range, representing an end-diastolic phase; and a second range, representing an end-systolic phase. In this way, the proposed method is able to identify whether or not the four-chamber image was captured during an end-diastolic phase or a end-systolic phase.

In some examples, the plurality of predefined ranges further comprises a third range, representing a mid-systolic phase. Including a mid-systolic phase range provides additional granularity in cardiac cycle analysis. This enhanced temporal resolution allows for more detailed assessment of cardiac function, e.g., to provide a clinician with additional information for making a clinical assessment.

In some examples, the first range covers a lower range of values than the second range. This recognizes that the relative size of the atria, with respect to the size of the ventricles, will be greater during an end-systolic phase.

In some examples, the atrial measure is a measure of the area of the atria of the heart; and the ventricle measure is a measure of the area of the ventricles of the heart. This provides a comprehensive representation of chamber sizes. This approach captures the full extent of chamber expansion and contraction, offering a more complete picture of cardiac function than linear measurements alone (e.g., diameters or the like)

In some examples, the method further comprises generating a score for the four-chamber ultrasound image data by processing at least the atrial measure and the ventricle measure. Generating a score based on atrial and ventricle measures provides a quantitative assessment of image quality and cardiac function. This scoring system can aid in quality control of ultrasound acquisitions and potentially identify subtle cardiac abnormalities.

In some examples, the method further comprises obtaining, from a classifier, a confidence score indicating a confidence that the four-chamber ultrasound image data provides a four-chamber view of the heart of the subject, wherein generating the score for the four-chamber ultrasound image data comprises processing at least the confidence score, the atrial measure and the ventricle measure. Incorporating a confidence score from a classifier enhances the reliability of the analysis.

In some examples, generating the score for the four-chamber ultrasound image data comprises: determining, as a second ratio, a ratio between the atrial measure and the ventricle measure or vice versa; and processing at least the confidence score and the second ratio to generate the score for the four-chamber ultrasound image data.

In some examples, processing at least the confidence score and the second ratio comprises: normalizing the confidence score and the second ratio; and performing a weighted sum of the normalized confidence score and the normalized second ratio to generate the score for the four-chamber ultrasound image data.

Normalizing and weighting the confidence score and ratio enables flexible and customizable scoring. This method allows for fine-tuning of the scoring algorithm to prioritize different aspects of image quality and cardiac function, e.g., to adapt the scoring algorithm to the specific use-case scenario in which it is to be used.

In some examples, the method further comprises processing the four-chamber ultrasound image data to predict whether or not atrioventricular valves of the subject are visible in the ultrasound image data, wherein the score for the four-chamber ultrasound image data is responsive to the prediction of whether or not the atrioventricular valves of the subject are visible in the ultrasound image data. Incorporating atrioventricular valve visibility into the scoring process improves the assessment of image quality and anatomical completeness.

In accordance with a proposed approach, there is provided a computer program product comprising computer program code means which, when executed on a computing device having a processing system, cause the processing system to perform all of the steps of any herein proposed computer-implemented method according to any one of the preceding examples.

In accordance with a proposed approach, there is provided a processing system for processing four-chamber ultrasound image data, providing a four-chamber view of a heart of a subject. The processing system is configured to: process the four-chamber ultrasound image data to determine an atrial measure providing a measure of a size of atria represented in the four-chamber ultrasound image data; process the four-chamber ultrasound image data to determine a ventricle measure providing a measure of a size of ventricles represented in the four-chamber ultrasound image data; and process the atrial measure and the ventricle measure to predict a cardiac phase of a cardiac cycle of the subject represented by the four-chamber ultrasound image data.

In accordance with a proposed approach, there is provided an ultrasound imaging system. The ultrasound imaging system comprises an ultrasound probe for acquiring ultrasound image data of a subject, and the processing system for processing four-chamber ultrasound image data. The processing system is further configured to detect the four-chamber ultrasound image data in the ultrasound image data.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a workflow;
Fig. 2 illustrates a proposed method;
Fig. 3 illustrates another proposed method; and
Fig. 4 illustrates another proposed method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for processing four-chamber ultrasound image data. The image data is processed to predict a cardiac phase represented by the view by first identifying an atrial measure and a ventricle measure. These measures are subsequently processed to identify the cardiac phase.

The present disclosure relates to the identification of a cardiac phase represented in ultrasound image data, specifically four-chamber ultrasound image data. It is well known that a heart (comprising two atria and two ventricles) will iteratively perform a cardiac cycle to pump blood. The cardiac cycle is divisible into a number of phases, including a diastolic phase and a systolic phase. Of course, the cardiac cycle is divisible more granularly, e.g., into an end-diastolic phase, a mid-systolic phase, an end-systolic phase and a mid-diastolic phase.

The end-diastolic phase represents the point just before ventricular contraction, when the ventricles are at their fullest. The mid-systolic phase occurs during ventricular contraction, while the end-systolic phase is the point of maximum contraction, just before the ventricles begin to relax. The mid-diastolic phase is the period of ventricular filling between atrial contraction and the next ventricular contraction.

A number of proposed embodiments make use of a machine-learning method to perform a function or task. A machine-learning method is any self-training algorithm that processes input data in order to produce or predict output data.

Suitable machine-learning methods for being employed in the present invention will be apparent to the skilled person. Examples of suitable machine-learning methods include decision tree algorithms and artificial neural networks. Other machine-learning methods such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

A decision tree algorithm processes input data through a tree of nodes. In the tree of nodes, each successive node splits into two or more further nodes until reaching a terminal or end node. When performing the decision tree algorithm using the tree of nodes, at each node, a decision is made as to which further node to move to next based on the input data. The end node defines the outcome of the decision tree algorithm, and therefore the machine-learning method.

Methods of training a machine-learning method are well known. Typically, such methods comprise obtaining a training dataset, comprising training input data entries and corresponding training output data entries.

For some machine-learning methods, such as a neural network, training is performed by applying an initialized machine-learning method to each input data entry to generate predicted output data entries. An error between the predicted output data entries and corresponding training output data entries is used to modify the machine-learning method. This process can be repeated until the error converges, and the predicted output data entries are sufficiently similar (e.g. ±1%) to the training output data entries. This is commonly known as a supervised learning technique.

For example, where the machine-learning method is formed from a neural network, (weightings of) the mathematical operation of each neuron may be modified until the error converges. Known methods of modifying a neural network include gradient descent, backpropagation algorithms and so on.

Other approaches for training machine-learning methods (e.g., decision trees) are known in the art. For instance, decision trees are often trained using a decision tree builder or learning techniques, such as those set out by Suthaharan, Shan, and Shan Suthaharan. "Decision tree learning." Machine Learning Models and Algorithms for Big Data Classification: Thinking with Examples for Effective Learning (2016): 237-269 or Ruggieri, Salvatore. "Yadt: Yet another decision tree builder." 16th IEEE International Conference on Tools with Artificial Intelligence. IEEE, 2004.

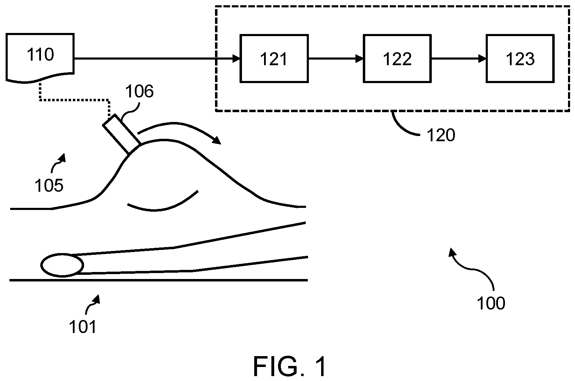

Fig. 1 schematically illustrates a workflow 100 into which a proposed method can be employed, for the purposes of improved contextual understanding. The workflow 100 represents the capture and processing of ultrasound image data.

Initially, ultrasound image data 110 is captured using an ultrasound imaging system 105. The ultrasound imaging system 105 is configured to use an ultrasound imaging technique to perform imaging of a subject 101 (e.g., a fetus) and capture ultrasound image data.

By way of working example, the ultrasound imaging system 105 may comprise an ultrasound probe 106 for acquiring ultrasound image data of the subject. More specifically, the ultrasound probe 106 may be an ultrasound transducer configured to produce ultrasound waves (which are directed into a subject) and measure reflected ultrasound waves (e.g., as reflected at tissue boundaries within the subject).

During an ultrasound scan, the ultrasound probe 106 is placed against the abdomen of a subject for imaging (e.g., of the subject or another subject carried by said subject, e.g., a fetus). The ultrasound probe 106 may produce a spread of ultrasound waves, each of which is projected (i.e., emitted) into the subject. A portion of each of the ultrasound waves is reflected at each tissue boundary encountered by the ultrasound wave. In particular, said reflected ultrasound waves are reflected back towards the ultrasound probe 106 where they are subsequently measured.

By processing the intensity (i.e., amplitude) and travel time of each of the measured (reflected) ultrasound waves, ultrasound image data representing the subject can be produced. Accordingly, by appropriately placing the ultrasound probe 106 on the subject, ultrasound image data comprising a representation of a subject can be acquired.

To provide further context, the previously mentioned travel time refers to the time interval between the ultrasound probe 106 emitting a wave of ultrasound and measuring/receiving a reflected wave of ultrasound. Assuming a constant speed of sound, the travel time for a reflected ultrasound wave is thus indicative of a depth (below the surface of the subject's abdomen) of a tissue boundary from which the ultrasound wave was reflected. Additionally, the amplitude of the measured ultrasound wave corresponds to the proportion of the initial ultrasound wave that was reflected by the tissue boundary. Accordingly, the ultrasound image data can be produced by populating pixels or voxels of a dataset based on the measured ultrasound waves, e.g., for each measured ultrasound wave, populating a specific pixel/voxel (based on the travel time of the wave and a detector channel of the ultrasound probe 106 that measured the wave) with an intensity/value proportional to the amplitude of the measured wave. This method of ultrasound imaging is better known as brightness-mode (i.e., B-mode) imaging.

During the ultrasound scan, the ultrasound probe 106 may also be moved relative to the subject while continuously emitting (and measuring) ultrasound waves. More particularly, the ultrasound probe 106 may be swept (i.e., translated) along a trajectory (i.e., path) on the surface of the subject's abdomen. A series/sequence of instances of ultrasound image data may thereby be acquired along this trajectory, where each instance in the sequence corresponding to a respective position of the ultrasound probe 106 along the trajectory.

The ultrasound image data (or each instance of ultrasound image data) 110 captured by the ultrasound imaging system 105 may be processed by a processing system 120. In some embodiments, the processing system 120 is configured to process the ultrasound image data to detect four-chamber ultrasound image data in the ultrasound image data 110.

The processing system 120 may comprise a detection module 121. The detection module is configured to process (each instance of) the ultrasound image data 110 to predict whether or not the (instance of) ultrasound image data 110 contains a representation of the heart of the subject.

The detection module 121 may perform this task by, for instance, processing (the instance of) ultrasound image data using a classification model, such as an object detection and/or segmentation model/algorithm. Such models are capable of identifying representations of a target anatomy (e.g., the heart) in image data (e.g., an ultrasound frame) based on the presence of certain shapes and/or spectral features that resemble an expected appearance for different cross-sections of the target anatomy.

Preferably, if used, the model may be (or form part of) a machine-learning model (e.g., a neural network, a transformer, etc.) trained to identify representations of the heart of a fetus in ultrasound frames.

Example machine-learning methods have been previously described, and are suitable for operation as a machine-learning model. For the purposes of training such a machine-learning method for this use, the training input data entries correspond to example classifications.

One specific suitable example of an appropriate model is the "You-only-look-once" (YOLO) model, e.g., described by Redmon, Joseph, et al. "You only look once: Unified, real-time object detection." Proceedings of the IEEE conference on computer vision and pattern recognition (2016).

Other suitable examples are well known to the skilled person.

The processing system 120 may also comprise a view classification module 122. The view classification module 122 is configured to process the ultrasound image data (only when it is predicted that the ultrasound image data contains a representation of the heart) to classify the view of the heart provided by the ultrasound image data. In this way, the view classification module 122 functions as a classifier for classifying the ultrasound image data.

In particular, the view classification module 122 is configured to determine at least whether or not the ultrasound image data contains a four-chamber view of the heart, i.e., whether or not the ultrasound image data is four-chamber ultrasound image data. Thus, the view classification module functions as a classifier configured to classify at least whether or not ultrasound image data provides a four-chamber view of the heart.

In this context, a four-chamber view of the heart is view that contains, represents or displays all four chambers of the heart (simultaneously). Thus, a four-chamber view will depict the right atrium, left atrium, right ventricle, and left ventricle in a single instance of ultrasound image data.

In some further examples, the view classification module 122 may be configured to determine which (if any) of a plurality of predetermined views of the heart is provided by the ultrasound image data. The plurality of predetermined views includes at least the four-chamber view, and may comprise one or more of the following: a left ventricular outflow tract (LVOT) view; a right ventricular outflow tract (RVOT) view; a three vessel view (3VV); and/or a three vessel and trachea (3VT) view. If the view classification module does not identify any view, this may be its own classification (e.g., labelled an OH view).

In some examples, the view classification module 122 is configured to employ an appropriately trained machine-learning method to process and classify the ultrasound data. In particular, the machine-learning network may be designed to distinguish between different standard cardiac views, such as those listed above.

Example machine-learning methods have been previously described, and are suitable for use by the view classificaiton module 122 to perform its function or task. For the purposes of training such a machine-learning method for this use, the training input data entries correspond to example instances of ultrasound image data (e.g., only instances of ultrasoudn image data containing a rerpesentation of the heart) and the training input data entries correspond to classifications indicating at least whether or not each example instance of ultrasound image data provides a four-chamber view.

In some examples, the view classification module may use a feature extraction algorithm to identify key anatomical landmarks within the ultrasound image data. These landmarks may include the left and right ventricles, left and right atria, and interventricular septum. The presence and spatial arrangement of these landmarks may be used to determine whether or not the ultrasound sound image represents a four-chamber view.

As another example, the view classification module may use a rule-based system that analyzes the shape, size, and relative positions of cardiac chambers visible in the ultrasound image data. For instance, it may check for the presence of four distinct chambers arranged in a specific configuration to predict whether or not the ultrasound image data provides a four-chamber view.

In some examples, the view classification module 122 may output a confidence score along with its classification (i.e., indicating at least whether or not the ultrasound image data provides a four-chamber view).

This confidence score may provide a quantitative measure of how certain the classifier is about its prediction. For instance, when classifying an image as a four-chamber view, the module may not only output the classification but also a confidence score, such as a value between 0 and 1, where 1 indicates the highest confidence and 0 the lowest.

The ability to output a confidence score alongside a classification is a well-known characteristic or capability of many classifiers, particularly those that employ appropriately trained machine-learning methods. As such, approaches for producing a confidence score are not described in detail. Nonetheless, purely by way of example, in a neural network-based classifier, the confidence score may be derived from the softmax output of the final layer. In a decision tree or random forest classifier, it may be based on the proportion of trees that agree on the classification.

The output of the view classification module is post-classification ultrasound image data, which contains at least one instance of four-chamber ultrasound image data.

The post-classification ultrasound image data may be further processed by a processing module 125 of the processing system 120. Alternatively or additionally, the processing system can be configured to receive four-chamber ultrasound image data. In other words, the detection module 121 and the view classification module 122 can be optional. Ultrasound image data 110 can be pre-processed by other processing system(s). The other processing system(s) can be part of the ultrasound imaging system or part of any imaging processing system.

The present disclosure relates to a mechanism for processing four-chamber ultrasound image data. Thus, the proposed mechanism may be employed by the processing module 125 of the processing system for processing (each instance of) four-chamber ultrasound image data. This allows for real-time or post-acquisition analysis of four-chamber views of the heart.

In particular, the present disclosure proposes approaches that facilitate identification of a cardiac phase represented in four-chamber ultrasound image data. The proposed method exploits the unique characteristics of four-chamber ultrasound image data to determine the cardiac phase represented in each instance (of four-chamber ultrasound image data). In particular, it has been identified that appropriate analysis of the relative sizes and shapes of the atria and ventricles, enables accurate prediction on whether the image represents end-diastole, end-systole, or intermediate phases of the cardiac cycle.

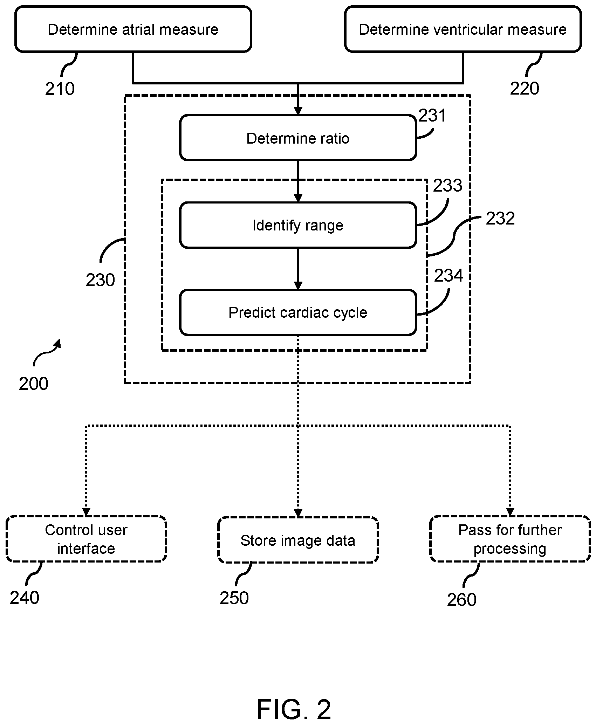

Fig. 2 is a flowchart illustrating a proposed computer-implemented method 200 for processing four-chamber ultrasound image data. The method 200 may be performed by the processing system 120 (Fig. 1), e.g., the processing module 125 of the processing system.

The method 200 comprise a step 210 of processing the four-chamber ultrasound image data to determine an atrial measure providing a measure of a size of atria represented in the four-chamber ultrasound image data.

By way of example, the atrial measure may be calculated as the combined area or volume of both atria. As another example, the atrial measure may be calculated as the sum of the circumferences of both atria. As another example, the atrial measure may be calculated as the sum of the diameters of both atria.

The method 200 also comprises a step 220 of processing the four-chamber ultrasound image data to determine a ventricle measure providing a measure of a size of ventricles represented in the four-chamber ultrasound image data.

Similarly, the ventricle measure may be calculated as the combined area or volume of both ventricles. As another example, the ventricle measure may be calculated as the sum of the circumferences of both ventricles. As another example, the ventricle measure may be calculated as the sum of the diameters of both ventricles.

A first approach to performing steps 210 and 220 is hereafter described.

In this first approach steps 210 and 220 may comprise processing the four-chamber ultrasound image data using one or more segmentation algorithms configured to segment the four-chamber ultrasound image data to identify portions representing the atria and the ventricles of the subject.

A wide variety of example approaches for performing such segmentation techniques are known in the art, such as those disclosed by Habijan, Marija, et al. "Overview of the whole heart and heart chamber segmentation methods." Cardiovascular engineering and technology 11 (2020): 725-747.

For the first approach, once the atria and the ventricles have been segmented in the four-chamber ultrasound image data, steps 210 and 220 may comprise determining the atrial measure and ventricle measure by calculating the area, volume, or other dimensional characteristics (e.g., summed diameters and/or circumferences) of the appropriate segmented regions.

A second example approach for performing steps 210 and 220 is hereafter described.

In this second approach, instead of using segmentation algorithms, steps 210 and 220 may use one or more feature detection techniques to identify one or more key anatomical landmarks within the four-chamber ultrasound image data. These landmarks may include the edges of the atrial and ventricular chambers, and optionally interventricular and interatrial septa.

Once these landmarks are identified, steps 210 and 220 may comprise calculating the atrial and ventricular measures using geometric approximations. For instance, the atrial measure could be estimated by fitting ellipses to the detected atrial boundaries and calculating their areas/volumes. Similarly, the ventricular measure could be determined by fitting ellipses to the detected ventricular boundaries and calculating their areas/volumes.

A third example approach for performing steps 210 and 220 is hereafter described. In this third approach, steps 210 and 220 comprise processing the four-chamber ultrasound image data using an appropriately trained machine learning model, which has been trained to directly estimate the atrial and ventricular measures from the four-chamber ultrasound image data. More particularly, the machine learning model may be designed to process the raw four-chamber ultrasound image data and output numerical values corresponding to the atrial and ventricular measures without the need for explicit segmentation or feature detection steps.

A wide variety of other suitable approach and techniques for performing steps 210 and 220 will be readily apparent to the appropriately skilled person.

The method 200 also comprises a step 230 of processing the atrial measure and the ventricle measure to predict a cardiac phase of a cardiac cycle of the subject represented by the four-chamber ultrasound image data.

Fig. 2 illustrates one example approach for performing step 230.

In this example, step 230 comprises a sub-step 231 of determining a ratio between the atrial measure and the ventricle measure; and a sub-step 232 of processing the determined ratio to predict the cardiac phase of the cardiac cycle of the subject represented by the four-chamber ultrasound image data.

This approach recognizes that the determined ratio (i.e., the atrial:ventricular ratio) varies predictably throughout the cardiac cycle. This allows for reliable and accurate identification of the cardiac phase.

For instance, during the end-diastolic phase, the ventricles are at their fullest, resulting in a lower atrial-to-ventricle ratio. Conversely, during the end-systolic phase, the ventricles are most contracted, leading to a higher atrial-to-ventricle ratio. Thus, appropriate analysis of the ratio allows for identification or prediction of the cardiac phase represented in the image.

In one approach, as illustrated, step 230 comprises a sub-step 231 of determining a ratio between the atrial measure and the ventricle measure (i.e., the atrial-to-ventricle ratio). This ratio may be calculated by dividing the atrial measure by the ventricle measure, or vice versa. The resulting ratio provides a quantitative representation of the relative sizes of the atria and ventricles at the moment the ultrasound image data was captured.

In this approach, step 230 may also comprise a sub-step 232 of processing the determined ratio to predict the cardiac phase of the cardiac cycle of the subject represented by the four-chamber ultrasound image data.

In particular, sub-step 232 may comprise comparing the determined ratio against predefined threshold values or ranges that correspond to different phases of the cardiac cycle. For instance, a ratio falling within a specific range might indicate an end-diastolic phase, while a ratio in a different range could suggest an end-systolic phase. These predefined ranges are typically established based on statistical analysis of a large number of cardiac ultrasound images and their corresponding known cardiac phases.

One approach for performing sub-step 232 is illustrated in Fig. 2.

In this approach, sub-step 232 comprises a sub-step 233 of identifying which of a plurality of predefined ranges bounds the determined ratio. In other words, sub-step 233 comprises determining which predefined range the calculated ratio falls within.

In this approach, sub-step 232 also comprises a sub-step 234 of predicting the cardiac phase of the cardiac cycle of the subject responsive to the identified predefined range.

It will therefore be appreciated that each predefined range corresponds to a specific phase of the cardiac cycle, such as end-diastole, end-systole, or mid-systole. By determining which range the calculated atrial-to-ventricle ratio falls into, it is possible to infer the most likely cardiac phase of the heart at the moment the ultrasound image data was captured.

For example, if the ratio falls within the range associated with end-diastole, sub-step 234 would predict that the four-chamber ultrasound image data represents the heart in its end-diastolic phase. Similarly, if the ratio falls within the range for end-systole, the prediction would be that the image captures the heart at end-systole.

Accordingly, the plurality of predefined ranges may comprise at least a first range, representing an end-diastolic phase; and a second range, representing an end-systolic phase. The plurality of predefined ranges may also comprise a third range, representing a mid-systolic phase.

It will be appreciated that the relationship between each predefined range and the cardiac phase may depend on how the ratio is calculated.

Consider a first scenario in which the ratio is defined by the atrial measure divided by the ventricle measure. In this case, a higher ratio may indicate the end-systolic phase, while a lower ratio may suggest the end-diastolic phase. Accordingly, the first range may have lower values than the second range.

In particular, for this first scenario, a higher ratio may indicate the end-systolic phase, while a lower ratio may suggest the end-diastolic phase. This is because during end-systole, the ventricles are contracted, resulting in a smaller denominator and thus a larger ratio. Conversely, during end-diastole, the ventricles are filled, leading to a larger denominator and a smaller ratio.

In a second scenario, the ratio is defined by the ventricle measure divided by the atrial measure. In this case, the relationship is reversed. Thus, a higher ratio may indicate the end-diastolic phase, while a lower ratio may suggest the end-systolic phase. This is because during end-diastole, the ventricles are filled, resulting in a larger numerator and thus a larger ratio. During end-systole, the ventricles are contracted, leading to a smaller numerator and a smaller ratio.

In this second scenario, the first range may have higher values than the second range.

In either scenario, the third range (if present) lies between the first range and the second range.

In some examples, the predefined ranges may be designed to cover all possible values of the atrial-to-ventricle ratio. This approach ensures that every calculated ratio can be associated with a cardiac phase prediction.

For example, the ranges include a first range that covers all values below a first threshold value. A second range may cover values from the first threshold value up to but not including a second threshold value. A third range may cover all values greater than or equal to the second threshold value.

In this way, the ranges are contiguous and cover all possible positive real numbers, ensuring that any calculated ratio will fall within one of the defined ranges. The use of open-ended ranges allows for accommodation of extreme or unexpected ratio values without leaving any gaps in the classification system.

It will be appreciated that the specific threshold values separating these ranges may be determined through statistical analysis of a large dataset of labelled cardiac ultrasound images. The threshold value(s) may be defined or responsive to one or more properties of the subject, such as subject age, heart size, or other relevant parameters.

As another example, step 230 may be performed by, for instance, normalizing the atrial measure and the ventricle measure. This normalization may be performed with respect to the size of the overall heart or the combined size of the atria and the ventricles.

A difference between the normalized sizes may be used to a similar effect as the ratio between the normalized atrial and ventricular sizes to predict the cardiac phase.

In this approach, predefined ranges may be established for the normalized difference between atrial and ventricular sizes. These ranges may correspond to different phases of the cardiac cycle. For instance, a range of larger positive differences may indicate the end-systolic phase, where the atria are relatively larger compared to the contracted ventricles. A range of smaller differences (close to zero) may suggest a mid-cycle phase, where the atria and ventricles are more similar in size. A range of larger negative differences may correspond to the end-diastolic phase, where the ventricles are relatively larger due to being filled with blood.

In this technique, step 230 may comprise calculating the normalized difference (as previously explained), then identifying which predefined range this difference falls into. The cardiac phase associated with that range may then be assigned to the four-chamber ultrasound image data.

Other suitable approaches for performing step 230 will be apparent to the appropriately skilled person.

The method 200 may further comprise a step 240 of controlling a user interface to provide a user-perceptible output representing the identified cardiac phase.

By way of example, step 240 may comprise controlling a display to present a visual indicator of the identified cardiac phase, e.g., alongside or overlaid on a visual representation of the four-chamber ultrasound image data. This visual indicator may take various forms, such as a text label, a color-coded marker, or an animated icon representing the current phase of the cardiac cycle.

In some examples, step 240 may comprise controlling an audio output device to provide an audible indication of the identified cardiac phase. This audible indication may include spoken words describing the phase, a distinct tone or series of tones corresponding to different phases, or a change in pitch or volume to represent different phases of the cardiac cycle.

The method 200 may comprise a step 250 of storing the four-chamber ultrasound image data with cardiac phase information (e.g., in the form of a flag and/or other identifier) indicating the identified cardiac phase. This storage step 250 allows for efficient organization and retrieval of the ultrasound image data based on the cardiac phase it represents.

The cardiac phase information can be implemented in various ways, such as metadata tags, file naming conventions, or database entries. For example, the image data could be stored with a filename suffix like "_ED" for end-diastole or "_ES" for end-systole. Alternatively, a metadata tag could be added to the image file itself, containing information about the identified cardiac phase. This approach facilitates quick filtering and sorting of stored images based on cardiac phase, which can be particularly useful for subsequent analysis, comparison of images across different phases, or for educational purposes.

Additionally, storing the cardiac phase information alongside the image data ensures that this crucial context is not lost, even if the images are transferred between different systems or reviewed at a later date by different clinicians.

In some examples, as later described, the four-chamber ultrasound image data is only stored when one or more predetermined storage criteria have been met.

The method 200 may comprise a step 260 of passing the identified cardiac phase (and optionally the four-chamber ultrasound image data) to another algorithm or processing module to perform further processing.

This step enables the integration of the cardiac phase information into broader analytical workflows or diagnostic processes. For instance, the identified cardiac phase could be used to trigger specific measurements or analyses that are phase-dependent, such as ejection fraction calculations during end-systole and end-diastole.

Exemplary further processing steps that may be performed may include determining one or more functional parameters (e.g., end-diastole volume, end-systole volume, sphericity index and strain), calculating cardiac output (e.g., ejection fraction), assessing ventricular function, or detecting wall motion abnormalities.

The identified cardiac phase may also be used to guide the selection of appropriate reference ranges for various cardiac measurements, as normal values often differ between end-systole and end-diastole. Additionally, the cardiac phase information may be utilized in image registration algorithms for comparing multiple ultrasound images or for aligning ultrasound data with other imaging modalities.

A wide variety of other uses for the identified cardiac phase (i.e., the cardiac phase information) will be readily apparent to the appropriately skilled person.

In some examples, step 260 may comprise performing the further processing on the identified cardiac phase (and optionally the four-chamber ultrasound image data).

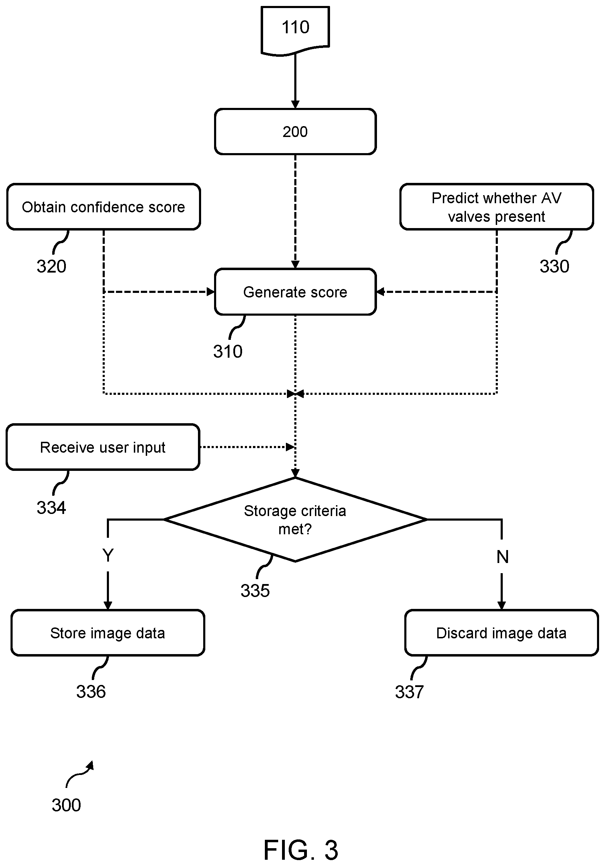

Fig. 3 is a flowchart illustrating another proposed computer-implemented method 300 for processing four-chamber ultrasound image data. The method 300 may be performed by the processing system 120 (Fig. 1), e.g., the processing module 125 of the processing system.

The method 300 comprises performing method 200 (Fig. 2) previously disclosed. The method 300 also comprises one or more optional steps, which are identified below.

The method 300 may also comprises a step 310 of generating a score for the four-chamber ultrasound image data by processing at least the atrial measure and the ventricle measure.

This scoring step 310 effectively provides a quantitative assessment of the four-chamber ultrasound image data, taking into account the relative sizes of the atria and ventricles. The score can serve multiple purposes, including evaluating image quality, assessing cardiac function, or providing an indication of clinical usefulness of the four-chamber ultrasound image data.

In particular, the score generated in step 310 may be at least partially dependent upon the identified cardiac phase of the four-chamber ultrasound image data. This allows the scoring system to consider the dynamic nature of the heart's cycle when evaluating the image data, and in particular the recognition that different properties of the image data would be advantageous for different phases of the cardiac cycle.

In some examples, method 300 comprises obtaining 320, from a classifier (such as the view classification module 122 (Fig. 1)), a confidence score indicating a confidence that the four-chamber ultrasound image data provides a four-chamber view of the heart of the subject. The confidence score likely reflects how certain the classifier is that the image truly represents a four-chamber view. A higher confidence score would indicate a clearer, more definitive four-chamber view, which could positively influence the overall score.

In such examples, step 310 may comprise processing at least the confidence score, the atrial measure and the ventricle measure to generate the score.

In particular, step 310 may comprise normalizing the confidence score and the second ratio; and performing a weighted sum of the normalized confidence score and the normalized second ratio to generate the score for the four-chamber ultrasound image data.

As a working example, the score S_{ED} for an instance of four-chamber ultrasound image data identified as representing an end-diastolic phase may be calculated as: ${S}_{ED} = X . CS + \left(1-X\right) . ED$ wherein X is a first weighting between 0 and 1, CS is the confidence score for the four-chamber ultrasound view and ED is an end-diastole score, which may be calculated as: $ED = 100 \left(1-A: V\right)$ where A is the atrial measure and V is the ventricle measure (for said four-chamber ultrasound image data). In this way, the end-diastole score is effectively calculated using a second ratio, being a ratio between the atrial measure and the ventricle measure (or vice versa).

By way of example, the value of X may range from between 0.5 and 0.6.

In this working example, the score S_{ES} for an instance of four-chamber ultrasound image data identified as representing an end-systolic phase may be calculated as: ${S}_{ES} = Y . CS + \left(1-Y\right) . \left(1-ED\right)$ where Y is a second weighting between 0 and 1. By way of example, the value of Y may range from between 0.5 and 0.6.

In some examples, method 300 further comprises processing 330 the four-chamber ultrasound image data to predict whether or not atrioventricular valves of the subject are visible in the ultrasound image data. The atrioventricular valves, which include the mitral valve (between the left atrium and left ventricle) and the tricuspid valve (between the right atrium and right ventricle), are well-known anatomical structures in the heart.

Ultrasound image data containing a user-visible representation of the atrioventricular valves of the subject is known as bookmark image data, as they contain a representation of important anatomical structures for analysis by a clinician.

In general, bookmark image data is often saved separately or flagged within the overall ultrasound system for easy retrieval and review. They serve as reference points for the clinician, allowing access to the clinically useful image data. In the context echocardiography, these bookmark images with visible atrioventricular valves may be used for more detailed measurements, assessment of cardiac function, or discussion with other healthcare professionals.

Approaches for performing step 330 are well known in the art, such as edge detection algorithms, shape analysis techniques or machine learning models trained to recognize valve structures in ultrasound images.

In particular, machine learning models trained to recognize valve structures in ultrasound images can be particularly effective for performing step 330. These models are typically trained on large datasets of labeled ultrasound images, where the atrioventricular valves have been manually identified by experts. Convolutional neural networks (CNNs) are often employed for this purpose due to their ability to learn spatial hierarchies of features from image data.

In addition to binary classification (valve visible or not), some models are able to provide a confidence score for valve visibility ("bookmark score"). Other forms of valve identification algorithms may similarly produce a confidence score. For instance, traditional computer vision techniques using edge detection and shape analysis may assign confidence scores based on the strength of detected edges or the degree of match to predefined valve shapes.

In some examples, if performed, step 310 may use the confidence score for valve visibility (i.e., the bookmark score) to determine a score for the four-chamber ultrasound image data if one or more predetermined criteria are met.

For instance, in some examples, the bookmark score may be used in step 310 when the cardiac phase associated with the four-chamber ultrasound image data is a mid-systolic phase.

For instance, in one working example, the score S_{MS} for an instance of four-chamber ultrasound image data identified as representing a mid-systolic phase may be calculated as: ${S}_{MS} = Z . CS + \left(1-Z\right) . BS$ where Z is a third weighting between 0 and 1 and BS is the bookmark score. By way of example, the value of Z may range from between 0.3 and 0.5.

In a working example, the score S_{ED} for an instance of four-chamber ultrasound image data identified as representing an end-diastolic phase may be calculated as: ${S}_{ED} = X 1 . CS + X 2 . ED + X 3 . BS$ wherein X1, X2, X3 are different first weightings between 0 and 1 (preferably where the sum of X1, X2 and X3 is 1). By way of example, the value of X1 may range from between 0.4 and 0.5, the value of X2 may range from between 0.3 and 0.4, and the value of X3 may range from between 0.1 and 0.3.

Similarly, the score S_{ES} for an instance of four-chamber ultrasound image data identified as representing an end-systolic phase may be calculated as: ${S}_{ES} = Y 1 . CS + Y 2 . \left(1-ED\right) + Y 3 . BS$ wherein Y1, Y2, Y3 are different first weightings between 0 and 1 (preferably where the sum of Y1, Y2 and Y3 is 1). By way of example, the value of Y1 may range from between 0.4 and 0.5, the value of Y2 may range from between 0.3 and 0.4, and the value of Y3 may range from between 0.1 and 0.3.

The method 300 may comprise saving 335 or storing the four-chamber ultrasound image data (e.g., in a memory or database) responsive to whether or not the four-chamber ultrasound image data meets one or more predetermined storage criteria.

In particular, the four-chamber ultrasound image data may be saved/stored in a step 336 responsive to the one or more predetermined storage criteria being met. Step 336 may be performed using the approach previously described for optional step 260 (Fig. 2). Conversely, if the one or more predetermined criteria are not met, the method 300 may move to step 337 of discarding the image data or flagging it for further review.

In some examples, the one or more predetermined storage criteria may include a first storage criterion that the prediction of whether or not atrioventricular valves are present in the four-chamber ultrasound image data matches a user indicated desire. In this way, steps 335, 336 and 337 effectively perform a function of automatically saving the four-chamber ultrasound image data responsive to a user's choice of with or without AV valves.

More specifically, the method 300 may comprise a step 334 of receiving a user input indicating whether or not a user/clinician desires the atrioventricular (AV) valves of the subject to be visible in the ultrasound image data (i.e., the user-indicated desire).

The user input may be received through various interfaces or mechanisms integrated into the ultrasound imaging system or associated software. These may include a graphical user interface with selectable options for indicating atrioventricular valve visibility preferences, a touchscreen display for tapping or swiping selections, voice command functionality for verbal input, a physical control panel with dedicated buttons or dials, customizable presets for saved settings, prompts at the start of an exam to specify preferences, or remote input options from separate workstations or mobile devices on the system network.

Accordingly, the first storage criterion may be met when there is a match between the user indicated desire and the prediction produced in step 320.

In some examples, the one or more predetermined storage criteria may include a second storage criterion that the score (determined in method 200) is superior to a score for a previously stored instance of four-chamber ultrasound image data representing the same cardiac phase (e.g., for a same cardiac cycle and/or ultrasound imaging session). In some examples, one score is superior to another score when the score is higher.

Thus, the second storage criterion aims to prioritize the storage of higher quality four-chamber ultrasound image data.

Of course, if there is no previously stored instance of four-chamber ultrasound image data representing the same cardiac phase (e.g., for a same cardiac cycle and/or ultrasound imaging session), then the second storage criterion may be automatically met.

Other suitable examples of predetermined storage criteria will be apparent to the skilled person.

For instance, the predetermined storage criteria may include: a third storage criterion that the confidence score breaches a predetermined value; and/or a fourth storage criterion that the end-diastolic score falls within a desired range (which may differ dependent upon the identified cardiac phase) and so on.

In some examples, if there is a previously stored instance of four-chamber ultrasound image data representing the same cardiac phase (e.g., for a same cardiac cycle and/or ultrasound imaging session) and the one or more predetermined storage criteria are met, then step 336 may overwrite said previously stored instance. This is particularly advantageous when the one or more predetermined storage criteria include the second storage criteria, to effectively store the highest scoring image data.

In some examples, if performed, step 320 is dependent upon the user input received in step 334 and the outcome of step 330 (e.g., the confidence score produced in step 330). In particular, different algorithms may be used to generate the score for the four-chamber ultrasound image data responsive to whether or not the user input indicates that a user/clinician desires the atrioventricular valves of the subject to be visible in the ultrasound image data.

Put another way, the score for the four-chamber ultrasound image data may be responsive to the prediction of whether or not the atrioventricular valves of the subject are visible in the ultrasound image data.

This variation may be employed even when step 335 (and steps 336 and 337) are not employed in method 300 and/or the one or more predetermined storage criteria does not include the first storage criterion.

In some embodiments, the approach defined by equation (1) to (4), and previously described, are used when the user input indicates that the user/clinician does not desire the atrioventricular valves of the subject to be visible in the four-chamber ultrasound image data.

This approach recognizes that when the user does not desire visible atrioventricular valves, the scoring system should focus less upon the confidence that the ultrasound image data represents bookmark image data, and more on other aspects of the four-chamber view. In such cases, the scoring method prioritizes the confidence of the four-chamber view classification and the ratio between atrial and ventricular measures.

In some embodiments, a different set of equations may be used when the user input indicates that the user/clinician desires the atrioventricular valves of the subject to be visible in the four-chamber ultrasound image data. For instance, the approach defines by equations (4) to (6) may be used when the user input indicates that the user/clinician does desire the atrioventricular valves of the subject to be visible in the four-chamber ultrasound image data. It is noted that equation (4) may be used in either scenario.

It will be appreciated that method 200, 300 may be iteratively repeated, e.g., for each instance of four-chamber ultrasound image data identified in the workflow 100 (Fig. 1).

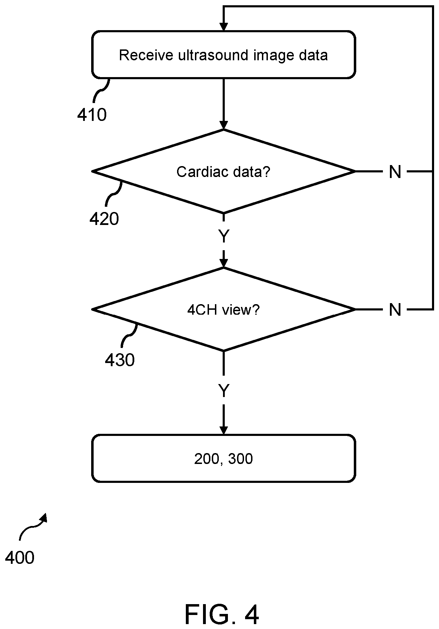

Fig. 4 is a flowchart illustrating another proposed computer-implemented method 400 for processing ultrasound image data. The method 400 may be performed by the processing system 120 (Fig. 1).

The method 400 comprises receiving ultrasound image data 410. Step 410 may comprise receiving the ultrasound image data from an ultrasound imaging system. In other examples, step 410 may comprise obtaining or capturing the ultrasound image data using the ultrasound image system.

Thus, step 410 may comprise a passive step (e.g., receiving or retrieving previously captured ultrasound image data) or an active step (e.g., actively capturing/generating the ultrasound image data).

The method 400 also comprises a step 420 of determining whether or not the ultrasound image data is cardiac ultrasound image data, e.g., contains a representation of the heart. This may be performed using any previously described approach, e.g., such as those described for describing the function of the detection module 121 (Fig. 2).

Responsive to a positive determination in step 420 (i.e., the ultrasound image data is identified as being cardiac image data), the method 400 performs step 430 of determining whether or not the ultrasound image data provides a four-chamber view of the heart. This may be performed using any previously described approach, such as those described for the function of the view classification module.

Responsive to a positive determination in step 430, the method 400 performs a previously described method 200, 300.

Responsive to a negative determination in step 410 or 420, the method 400 may revert back to step 410. This does not exclude the possibility of further processing steps being performed on the ultrasound image data before reverting back to step 410, which processing steps are immaterial to the underlying concept of the present disclosure.

In other examples, steps 420 and 430 are combined. Thus, the combined step 420, 430 may comprise directly identifying whether or not the ultrasound image data (obtained in step 410) provides a four-chamber view of a heart. This may be achieved using an appropriately trained machine-learning method.

The skilled person would be readily capable of developing a processing system for carrying out any herein described method. Thus, each step of the flow chart may represent a different action performed by a processing system, and may be performed by a respective module of the processing system.

In particular, the processing system 120 (Fig. 1) may be readily adapted or configured to perform the function of any herein described method.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method (200, 300, 400) of processing four-chamber ultrasound image data, providing a four-chamber view of a heart of a subject (101), the computer-implemented method comprising:
processing (210) the four-chamber ultrasound image data to determine an atrial measure providing a measure of a size of atria represented in the four-chamber ultrasound image data;
processing (220) the four-chamber ultrasound image data to determine a ventricle measure providing a measure of a size of ventricles represented in the four-chamber ultrasound image data; and
processing (230) the atrial measure and the ventricle measure to predict a cardiac phase of a cardiac cycle of the subject represented by the four-chamber ultrasound image data.

2. The computer-implemented method of claim 1, wherein the processing (230) the atrial measure and the ventricle measure comprises:
determining (231) a ratio between the atrial measure and the ventricle measure; and
processing (232) the determined ratio to predict the cardiac phase of the cardiac cycle of the subject represented by the four-chamber ultrasound image data.

3. The computer-implemented method of claim 2, wherein the processing (232) the determined ratio comprises:
identifying (233) which of a plurality of predefined ranges bounds the determined ratio; and
predicting (234) the cardiac phase of the cardiac cycle of the subject responsive to the identified predefined range.

4. The computer-implemented method of claim 3, wherein:
each predefined range is associated with a different phase of the cardiac cycle of the subject; and
the predicting the cardiac phase comprises identified the phase of the cardiac cycle associated with the identified predefined range.

5. The computer-implemented method of claim 3 or 4, wherein the plurality of predefined ranges comprises at least:
a first range, representing an end-diastolic phase; and
a second range, representing an end-systolic phase.

6. The computer-implemented method of claim 5, wherein the plurality of predefined ranges further comprises a third range, representing a mid-systolic phase.

7. The computer-implemented method of claim 5 or 6, wherein the first range covers a lower range of values than the second range.

8. The computer-implemented method of any one of claims 1 to 7, wherein:
the atrial measure is a measure of the area of the atria of the heart; and
the ventricle measure is a measure of the area of the ventricles of the heart.

9. The computer-implemented method (300, 400) of any one of claims 1 to 8, further comprising generating (310) a score for the four-chamber ultrasound image data by processing at least the atrial measure and the ventricle measure.

10. The computer-implemented method of claim 9, further comprising obtaining (320), from a classifier, a confidence score indicating a confidence that the four-chamber ultrasound image data provides a four-chamber view of the heart of the subject,
wherein generating the score for the four-chamber ultrasound image data comprises processing at least the confidence score, the atrial measure and the ventricle measure.

11. The computer-implemented method of claim 10, wherein generating the score for the four-chamber ultrasound image data comprises:
determining, as a second ratio, a ratio between the atrial measure and the ventricle measure or vice versa; and
processing at least the confidence score and the second ratio to generate the score for the four-chamber ultrasound image data.

12. The computer-implemented method of any one of claims 9 to 11, further comprising processing (330) the four-chamber ultrasound image data to predict whether or not atrioventricular valves of the subject are visible in the ultrasound image data,
wherein the score for the four-chamber ultrasound image data is responsive to the prediction of whether or not the atrioventricular valves of the subject are visible in the ultrasound image data.

13. A computer program product comprising computer program code means which, when executed on a computing device having a processing system, cause the processing system to perform all of the steps of the computer-implemented method according to any one of claims 1 to 12.

14. A processing system (120) for processing four-chamber ultrasound image data, providing a four-chamber view of a heart of a subject, wherein the processing system is configured to:
process (210) the four-chamber ultrasound image data to determine an atrial measure providing a measure of a size of atria represented in the four-chamber ultrasound image data;
process (220) the four-chamber ultrasound image data to determine a ventricle measure providing a measure of a size of ventricles represented in the four-chamber ultrasound image data; and
process (230) the atrial measure and the ventricle measure to predict a cardiac phase of a cardiac cycle of the subject represented by the four-chamber ultrasound image data.

15. An ultrasound imaging system (105), comprising:
an ultrasound probe (106) for acquiring ultrasound image data of a subject;
a processing system (120) of claim 14 for processing four-chamber ultrasound image data, providing a four-chamber view of a heart of the subject;
wherein the processing system (120) is further configured to detect the four-chamber ultrasound image data in the ultrasound image data.
